Europäisches Patentamt

European Patent Office

Office européen des brevets

⑩

(11) Publication number: **0 122 089**
**B2**

## (12) NEW EUROPEAN PATENT SPECIFICATION

(45) Date of publication of the new patent
specification: **12.12.90**

(21) Application number: **84302156.9**

(22) Date of filing: **29.03.84**

(51) Int. Cl.⁵: **A 61 B 1/12**

(54) **Endoscope.**

(30) Priority: **01.04.83 JP 58310/83**

(43) Date of publication of application:
**17.10.84 Bulletin 84/42**

(45) Publication of the grant of the patent:
**12.08.87 Bulletin 87/33**

(45) Mention of the opposition decision:
**12.12.90 Bulletin 90/50**

(84) Designated Contracting States:
**DE FR GB IT NL SE**

(56) References cited:
EP-A-0 028 825    DE-A-2 065 515
EP-A-0 037 047    DE-A-2 805 451
EP-A-0 062 315    DE-A-2 848 484
WO-A-82/03545    US-A-4 066 071
WO-A-83/03188

JP-A 55/151936
JP-A 141228
JP-A 57/126086

(73) Proprietor: **SUMITOMO ELECTRIC INDUSTRIES**
**LIMITED**
**No. 15, Kitahama 5-chome Higashi-ku**
**Osaka-shi Osaka 541 (JP)**

(72) Inventor: **Ono, Kimizo**
**Sumitomo Electric Ind. Ltd. 1-3, Shimaya 1-**
**chome**
**Konohana-ku Osaka (JP)**
Inventor: **Tsuno, Koichi**
**Sumitomo Electric Ind. Ltd. 1-3, Shimaya 1-**
**chome**
**Konohana-ku Osaka (JP)**
Inventor: **Nishikawa, Mitsuru**
**Sumitomo Electric Ind. Ltd. 1-3, Shimaya 1-**
**chome**
**Konohana-ku Osaka (JP)**

(74) Representative: **Rackham, Stephen Neil et al**
**GILL JENNINGS & EVERY 53-64 Chancery Lane**
**London WC2A 1HN (GB)**

EP 0 122 089 B2

Courier Press, Leamington Spa, England.

## Description

This invention relates to endoscopes, which may be used for example for industrial or medical applications for instance for observing an image of an area filled with an opaque liquid such as crude oil or blood.

Endoscopes for observing an image of a confined area filled with an opaque liquid such as the internal wall of the heart or a blood vessel filled with blood or the internal wall of a pipe filled with crude oil are known.

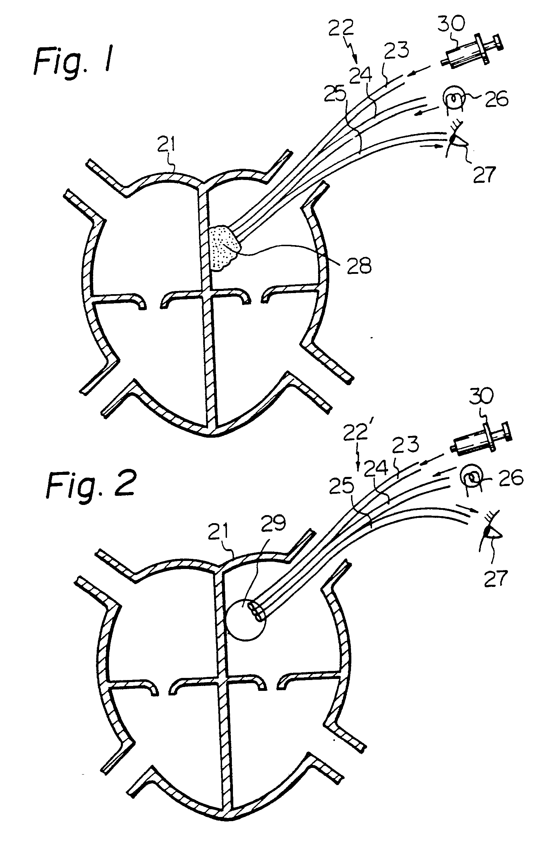

Figure 1 of the accompanying drawings is a schematic view showing the principle of the observation operation of the internal wall of the heart filled with blood by the use of a conventional endoscope. An endoscope 22 is inserted into the atrium of the heart 21. The endoscope comprises a transparent liquid supply tube 23, a light guide 24 and an image fibre 25, all of which are enclosed in a flexible tube. At the distal end of the image fibre a lens system may be provided so as to project the image of the object on the proximal end of the image fibre.

A transparent liquid is transported through the transparent liquid supply tube 23 and is flushed into the heart by a syringe 30. The light from a light source 26 is transmitted through the light guide 24 to illuminate the inside of the heart. By the flush of the transparent liquid 28 the blood around the end of the endoscope is displaced momentarily by the transparent liquid. At this moment, the internal wall of the heart makes its appearance in front of the leading end of the endoscope. The image of the illuminated internal wall is transmitted to an observer's eye 27 through the image fibre 25.

The above observation method, however, has the problem that observation time is too short. Namely, although an opaque liquid is displaced by the transparent flushing liquid, this occurs only momentarily. The transparent liquid and the opaque liquid mix with each other, the field of vision promptly being lost. In addition, in medical applications there is a risk of the flush of a transparent liquid into a blood vessel causing some harmful effect on the living body.

In order to resolve the above-mentioned problems, the Applicants have developed an endoscope which has a transparent balloon at its distal end. With this endoscope, a transparent liquid is injected into the balloon, and opaque liquid is displaced by the inflated balloon, and thus a field of vision is provided.

Figure 2 of the accompanying drawings shows this type of endoscope 22'.

The leading end of the transparent liquid supply tube 23 is not open but is covered by a transparent balloon 29 which also covers the distal ends of a light guide 24 and an image fibre 25.

At the opposite end of the transparent liquid supply tube 23, a syringe 30 for inflating the balloon with liquid is provided. A transparent liquid is forced into the tube 23 and is transported into the transparent balloon 29 by the syringe 30.

The balloon 29 is inflated to push away the surrounding opaque liquid.

By pressing the balloon 29 into contact with a surface to be observed, the opaque liquid that obscures the field of vision is cleared from the area between the observed surface and the leading end of the endoscope. Only the transparent liquid in the transparent balloon stays there. Therefore, it is made possible to obtain a bright and clear image of the observed surface. In addition, the displacement of the opaque liquid by the transparent liquid is not momentary. It is possible to continue the observation under the same conditions as long as the balloon is kept inflated.

The above-mentioned type of endoscope, however, still has the problem mentioned below.

It is important that endoscopes have small outer diameters. This is especially important with endoscopes for medical applications. For this reason, constituent members of the endoscope, for exmaple, the transparent liquid supply tube 23 which may be several metres long is restricted to an inner diameter of less than 0.5 mm.

When the endoscope is not in use, the transparent liquid supply tube 23 and the deflated balloon 29 are filled with air instead of the transparent liquid (a physiological solution of sodium chloride).

When the endoscope is in use, the transparent liquid is forced into the tube 23 and the balloon 29. Air inside the tube 23 and the balloon can be removed by alternate injection and discharge of the liquid. However, because there is only one liquid tube in the endoscope, the liquid in the tube must be sucked out in order to remove air bubbles. By the suction of the liquid, the volume of the liquid in the tube and the balloon is reduced gradually. Therefore the flow rate of the liquid is also reduced its air bubble carrying or entrainment capacity is gradually lowered. The result is that air bubbles remain on the internal wall of the tube and balloon. These air bubbles are difficult to remove due to the effect of their surface tension. In this way, air bubbles, although small in total volume, remain in the tube or balloon.

Air bubbles in the balloon prevent proper observation of the observed surface because they reflect or scatter the light. Therefore, it is necessary to remove air bubbles completely from inside the tube and balloon for effective observation. To prevent the generation of air bubbles, it is considered possible to remove air from the tube and balloon by a vacuum pump before the use of the endoscope and the supply of the liquid. This technique, however, requires a vacuum pump and is not easy to apply. In addition, it is difficult to remove air by suction all along the tube unless the tube is sufficiently rigid, because the injection liquid tube is long and yet very small in inner diameter.

The object of the present invention is to provide an endoscope which facilitates the removal of air from a transparent liquid duct and a balloon which form part of the endoscope without it being necessary to use a vacuum pump before the endoscope is used.

To this end, according to this invention, an endoscope for optical examination of a surface comprising an image fibre for transmitting an image of a surface to be examined in front of a distal end of the endoscope, a lens system for focussing an image of the surface to be examined onto the distal end of the image fibre, a light guide for transmitting light to the distal end of the endoscope to illuminate the surface to be observed, a liquid supply duct for the supply of transparent liquid, the image fibre, the light guide and the liquid supply duct being arranged generally side by side over part at least of their length and a balloon positioned to cover the lens system and the distal ends of the image fibre, the light guide and the liquid supply duct, so that the balloon can be inflated with a transparent liquid supplied through the liquid supply duct, is characterised by a liquid outlet duct extending beside the liquid supply duct over at least part of its length and having its distal end covered by the balloon, to enable transparent liquid to be supplied to and discharged from the ballon simultaneously to provide a flow of the liquid through the balloon, and in that the distal end of the endoscope includes a moulded endpiece covering the lens system, image fibre, light guide, and inlet and outlet tube, the end piece being sized to fit within an insertion tube and being surrounded by a sleeve of smaller cross section that the insertion tube, the balloon being fixed to the outside of the sleeve and the fixing and the free end of the ballon being covered by a band; the cross-section of the distal end being no greater than that of the insertion tube.

An endoscope constructed in accordance with the invention has the following advantages:

(1) Bubbles in the liquid which, in use, are in the transparent liquid passage and the balloon can be removed easily and effectively since the flow of the transparent liquid can be maintained at a constant rate by injecting a transparent liquid into the balloon through the inlet passage and discharging the transparent liquid through the outlet passage simultaneously.

(2) Since bubbles can be removed by the flow of the transparent liquid, it is not necessary to remove air from the transparent liquid passage and the balloon by a vacuum pump before the use of the endoscope.

An example of an endoscope in accordance with the invention will now be described with reference to the accompanying drawings, in which:

Fig. 1 as already mentioned is a schematic view showing the principles involved in the observation of the internal wall of a human heart using a conventional endoscope;

Fig. 2 is a schematic view showing the principles involved in the observation of the internal wall of the heart by the use of a known endoscope equipped with a balloon at its distal end;

Fig. 3 is a schematic view showing the whole arrangement of the endoscope of the present invention;

Fig. 4 is a perspective view of the distal end portion of the endoscope of Fig. 3;

Fig. 5 is an axial cross-sectional view corresponding to Fig. 4;

Fig. 6 is a cross-sectional view of the endoscope taken along the line VI—VI of Fig. 5;

Fig. 7 is a cross-sectional view of the endoscope taken along the line VII—VII of Fig. 5; and

Fig. 8 is a cross-sectional view of the endoscope taken along the line VIII—VIII of Fig. 5.

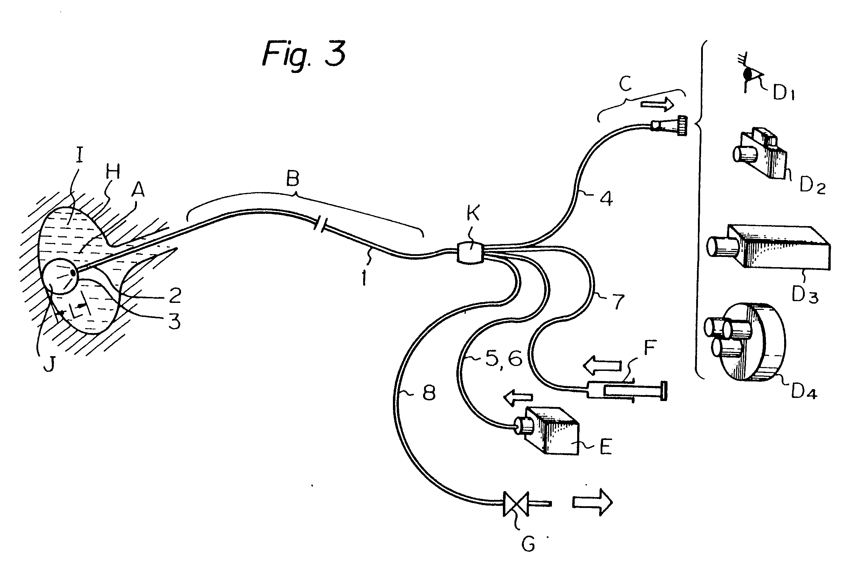

As shown in Fig. 3, the endoscope includes an image-sensing portion A for obtaining an image of an observed object, an image-observing portion C, and an image-transmitting portion B covered by a long flexible tube between the portions A and C.

At the image-observing portion C, the image of the observed object transmitted through an image fiber is received by an image-observing adapter. The received image can be observed in various ways. Namely, the image can be observed by the naked eye D1. The image can be photographed by a camera D2. The image can be observed on a monitor-TV through a TV camera D3. Or, the image can be recorded by a 16 mm motion picture camera D4.

At the end of the image-sensing portion A, a transparent balloon 3 is fixed to a flexible cable 1 by means of a sleeve 2.

The flexible cable 1 of the endoscope includes an image fiber 4 for transmitting an image, light guides 5, 6 for transmitting illumination light, an inlet tube 7 for transporting a transparent liquid into the balloon so as to inflate the balloon, an outlet tube 8 for discharging the transparent liquid and bubbles from inside the balloon, and an insertion tube 9 covering the above-mentioned members.

The flexible cable 1 branches off into the image fiber 4, light guides 5, 6, inlet tube 7 and outlet tube 8 at a parting portion K in the image-observing portion side.

The light guides 5, 6 are connected to an illumination light source E. The illumination light is transmitted along the transmitting portion B, and is emitted from the distal end of the image-sensing portion A. The emitted light illuminates the observed subject through a transparent liquid.

The inlet tube 7 is connected to a cylinder F for inflating the balloon.

The outlet tube 8 is connected to a discharging valve G.

Fig. 3 shows that the leading end of the image-sensing portion A has been inserted into the heart H and the balloon is inflated. The blood 1 is being pushed away by the balloon 3 which is filled by a transparent liquid J. The illumination light is emitted from the end of the image-sensing portion and illuminates the internal wall of the heart through the transparent liquid. The image of the internal wall is thrown on the end surface of the image fiber 4 through a lens system.

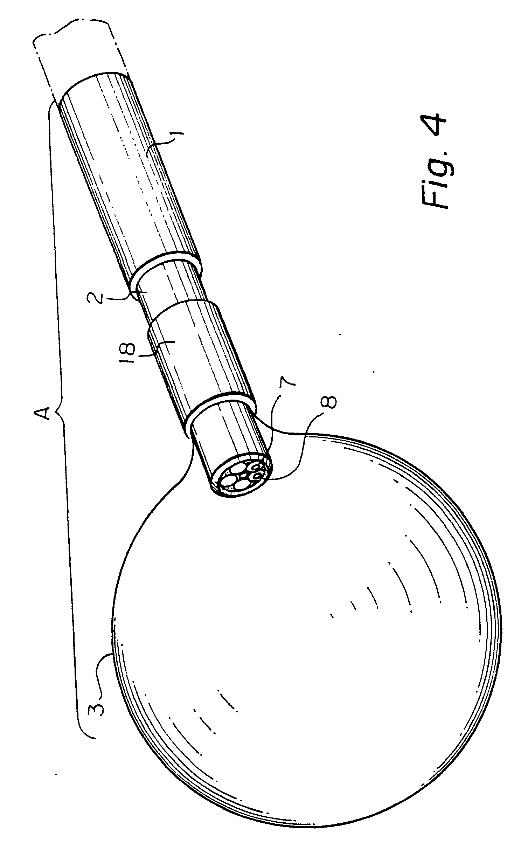

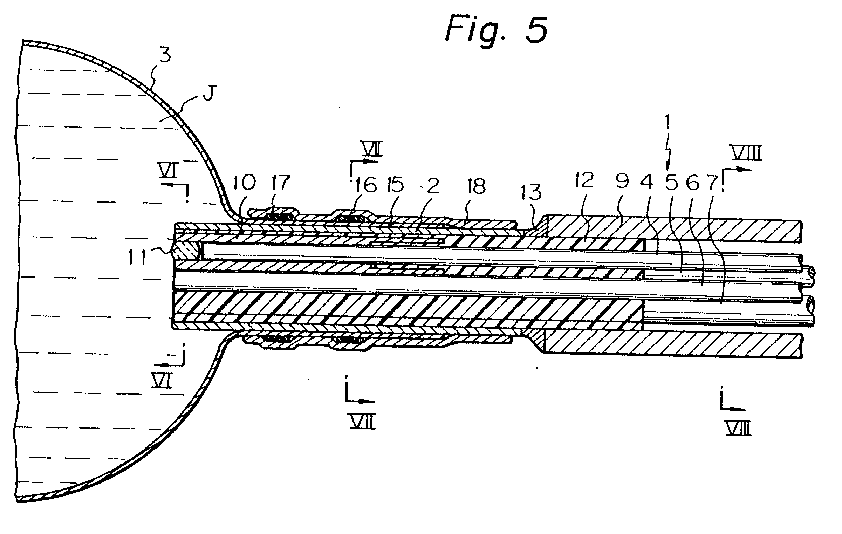

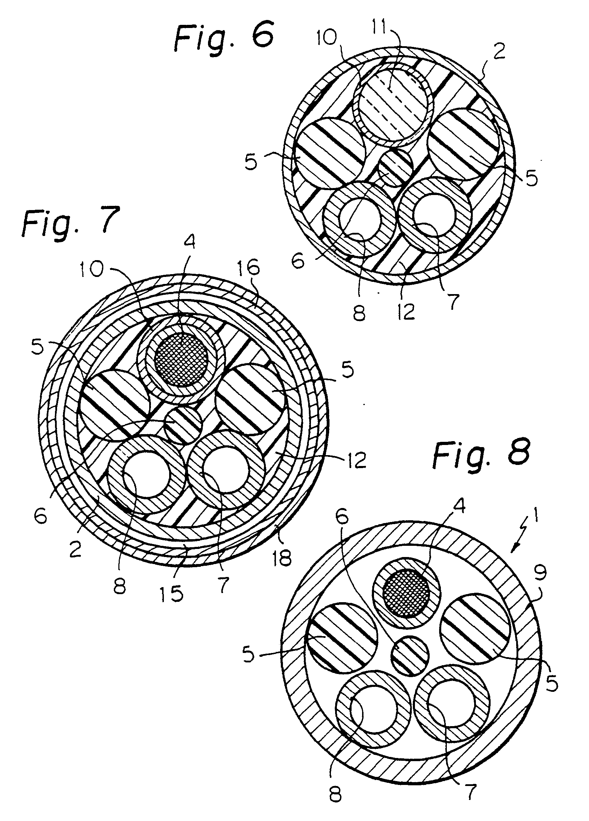

Fig. 4 is a perspective view of the image-sensing portion A of the endoscope. Fig. 5 is an enlarged cross-sectional view of the image-sensing portion. Figs. 6, 7 and 8 are cross-sectional views corresponding to lines VI—VI, VII—VII and VIII—VIII of Fig. 5, respectively.

The arrangement inside the flexible cable 1 is explained referred to Fig. 8.

The insertion tube 9 having an outer diameter of 2.8 mm and inner diameter of 2.2 mm contains six (6) fibers and tubes therein in this example.

A single image fiber 4 is located a little apart from the center of the cable.

Light guides 5, 5 are located symmetrically and a light guide 6 is located closer to the center of the cable. In this example, the light guide 5 has a diameter of 0.75 mm and the light guide 6 has a diameter of 0.5 mm. They can be made of a transparent plastic fiber such as PMMA (polimethyl methacrylate resin).

In addition, an inlet tube 7 and an outlet tube 8, both being of the same diameter, are located at symmetrical positions. Because two tubes are provided, this image fiber system can be called a two tube type. These tubes 7, 8 have an outer diameter of 0.6 mm and an inner diameter of 0.3 mm.

The structure of the image-catching portion A is explained referring to Fig. 5 through Fig. 7.

Around the leading end of the image fiber 4, a sleeve 10 is fixed. In front of the end surface of the image fiber 4, a micro lens 11 is fixed.

The distal end portions of the image fiber 4, light guide 5, 6, inlet tube 7 and outlet tube 8 are covered by a resin molded into a cylindrical shape. The molded resin 12 has a diameter of 2.0 mm and a length of 12 mm in this example.

The rear portion of the molded resin 12 is fitted into the insertion tube 9, and the sleeve 2 for supporting the balloon is fitted onto the front portion of the molded resin 12. The mouth portion 15 of the balloon 3 is fixed around the sleeve 2 by strings 16, 17 and a ring band 18. The sleeve 2, molded resin 12 an insertion tube 9 are bonded to each other by a binding agent 13.

In order to observe an object inside a human body, the flexible cable 1 of the endoscope is inserted into an organ inside the human body. At this time, the balloon 3 is kept in a fully deflated position. When the image-sensing portion A of the endoscope arrives at a desired position in the subject organ, the transparent liquid J is injected into the inlet tube 7 from the balloon inflation cylinder F. The transparent liquid J is transported along the the transmitting portion B into the balloon 3. The balloon 3 is inflated by the transparent liquid.

In order to remove air from inside the balloon 3 and tubes 7, 8, the discharge valve G is opened. The transparent liquid inflates the balloon 3 and is discharged through the outlet tube 8 and discharge valve G. Small bubbles in the tubes and balloon are carried out through the discharge valve G by the flow of the transparent liquid.

When the bubbles are all removed, the discharge valve G is closed. Except for the above-mentioned air-removing operation, the endoscope of the present invention can be operated in the same manner as the conventional endoscopes.

The desirable distance L between the observed surface and the distal end of the image-sensing portion is, for example, 10 mm. The balloon may be made of, for example, urethane rubber. When the balloon is inflated to 10 mm diameter, the amount of the transparent liquid, for example a physiological solution of sodium chloride, is 0.52 $cm^3$ in the balloon and 0.24 $cm^3$ total in the inlet and outlet tubes (each 1.7 m long).

## Claims

1. An endoscope for optical examination of a surface comprising an image fibre (4) for transmitting an image of a surface to be examined in front of a distal end of the endoscope, a lens system (11) for focussing an image of the surface to be examined onto the distal end of the image fibre (4), a light guide (5, 6) for transmitting light to the distal end of the endoscope to illuminate the surface to be observed, a liquid supply duct (7) for the supply of transparent liquid, the image fibre (4), the light guide (5, 6) and the liquid supply duct (7) being arranged generally side by side over part at least of their length and a balloon (3) positioned to cover the lens system (11) and the distal ends of the image fibre (4), the light guide (5, 6) and the liquid supply duct (7), so that the balloon (3) can be inflated with a transparent liquid supplied through the liquid supply duct (7), characterised by a liquid outlet duct (8) extending beside the liquid supply duct over at least part of its length and having its distal end covered by the balloon (3), to enable transparent liquid to be supplied to and discharged from the balloon simultaneously to provide a flow of the liquid through the balloon (3), and in that the distal end of the endoscope includes a moulded endpiece (12) covering the lens system (11), image fibre (4), light guide (5, 6), and inlet and outlet tube (7, 8), the end piece (12) being sized to fit within an insertion tube (9) and being surrounded by a sleeve (2) of smaller cross section than the insertion tube (9), the balloon (3) being fixed to the outside of the sleeve (2) and the fixing and the free end of the balloon (3) being covered by a band (18); the cross-section of the distal end being no greater than that of the insertion tube (9).

2. An endoscope according to claim 1, characterised in that the liquid supply duct (7) and the liquid outlet duct (8) are tubes of equal diameters.

3. An endoscope according to claim 1 or claim 2, characterised in that the outlet duct (8) is provided with a shut-off valve (G).

## Patentansprüche

1. Endoskop zur optischen Untersuchung einer Oberfläche mit einem ein Bild einer vor einem distalen Ende des Endoskops gebegenen, zu

**EP 0 122 089 B2**

untersuchenden Oberfläche übertragenden Glasfaserbündel (4), einem Linsensystem (11) zum Fokussieren eines Bildes der zu untersuchenden Oberfläche auf das distale Ende des Bildglasfaserbündels (4), einem Lichtleiter (5, 6) zu Übertragung von Licht zum distalen Ende des Endoskops zur Beleuchtung der zu untersuchenden Oberfläche, einem Flüssigkeitszufuhrschlauch (7) zur Zufuhr einer transparenten Flüssigkeit, wobei das Bildglasfaserbündel (4), der Lichtleiter (5, 6) und der Flüssigkeitszufuhrschlauch (7) über wenigstens einen Teil ihrer Länge nebeneinander angeordnet sind und ein Ballon (3) so angeordnet ist, daß er das Linsensystem (11) und die distalen Enden des Bildfaserbündels (4), des Lichtleiters (5, 6) und des Flüssigkeitszufuhrschlauches (7) derart abdeckt, daß der Ballon (3) mit einer durch den Flüssigkeitszufuhrschlauch (7) zugeführten transparenten Flüssigkeit aufblähbar ist, dadurch gekennzeichnet, daß über wenistens einen Teil der Länge des Flüssigkeitszufuhrschlauches (7) sich ein Flüssigkeitsabgabeschlauch (8) erstreckt, dessen distales Ende vom Ballon (3) abgedeckt ist, so daß unter Aufrechterhaltung einer Flüssigkeitsströmung innerhalb des Ballons (3) gleichzeitig die transparente Flüssigkeit zugeführt und von dem Ballon (3) abgegeben werden kann, und daß das distale Ende des Endoskops ein das Linsensystem (11), das Bildfaserbündel (4), den Lichtleiter (5, 6) und den Zufuhr- und Abgabeschlauch (7, 8) umgebendes Form-Endstück (12) enthält, dessen Abmessungen so gewählt sind, daß es in einen Mantelschlauch (9) paßt und das von einer Hülse (2) mit geringerem Querschnitt als der Mantelschlauch (9) umgeben ist, wobei der Ballon (3) an der Außenseite der Hülse (2) befestigt ist und die Befestigungsstelle und das freie Ballonende von einem Band (18) abgedeckt ist und wobei der Querschnitt des distalen Endes nicht größer als der des Mantelschlauches (9) ist.

2. Endoskop nach Anspruch 1, dadurch gekennzeichnet, daß der Flüssigkeitszufuhrschlauch (7) und der Flüssigkeitsabgabeschlauch (8) als Schläuche gleichen Durchmessers ausgebildet sind.

3. Endoskop nach Anspruch 1 oder 2, dadurch gekennzeichnet, daß der Flüssigkeitsabgabeschlauch (8) mit einem Absperrventil (G) versehen ist.

## Revendications

1. Endoscope pour l'examen optique d'une surface comprenant une fibre d'image (4) pour transmettre une image d'une surface à examiner devant une extrémité distale de l'endoscope, un système de lentilles (11) pour focaliser une image de la surface à examiner sur l'extrémité distale de la fibre d'image (4), un guide de lumière (5, 6) pour transmettre la lumière à l'extrémité distale de l'endoscope pour éclairer la surface à observer, une gaine (7) d'alimentation en liquide pour l'alimentation en liquide transparent, la fibre d'image (4), le guide de lumière (5, 6) et la gaine d'alimentation en liquide (7) étant agencés généralement côte-à-côte sur une partie au moins de leur longueur, et un ballon (3) placé pour couvrir le système de lentilles (11) et les extrémités distales de la fibre d'image (4), du guide de lumière (5, 6) et de la gaine d'alimentation en liquide (7) de manière que la ballon (3) puisse être gonflé d'un liquide transparent fourni à travers la gaine (7) d'alimentation en liquide, caractérisé par une gaine (8) de sortie du liquide qui s'étend à côté de la gaine d'alimentation en liquide sur au moins une partie de sa longueur et ayant son extrémité distale couverte par le ballon (3), pour permettre au liquide transparente d'être fourni et évacué du ballon simultanément en produisant un écoulement du liquide à travers le ballon (3) et en ce que l'extrémité distale de l'endoscope comporte un embout moulé (12) couvrant le système de lentilles (11), la fibre d'image (4) et le guide de lumière (5, 6) et le tube d'entrée et de sortie (7, 8), l'embout (12) étant dimensionné pour s'adapter dans un tube d'insertion (9) et étant entouré d'un manchon (2) de plus petite section transversale que le tube d'insertion (9), le ballon (3) étant fixé à l'extérieur du manchon (2) et la fixation et l'extrémité libre du ballon (3) étant couvertes d'une bande (18); la section transversale de l'extrémité distale n'étant pas plus grande que celle du tube d'insertion (9).

2. Endoscope selon la revendication 1, caractérisé en ce la gaine d'alimentation en liquide (7) et la gaine de sortie du liquide (8) sont des tubes de diamètres égaux.

3. Endoscope selon la revendication 1 ou la revendication 2, caractérisé en ce que la gaine de sortie (8) est pouvue d'une vanne d'arrêt (G).

Fig. 1

Fig. 2

Fig. 3

EP 0 122 089 B2

*Fig. 4*

Fig. 5

*Fig. 6*

*Fig. 7*

*Fig. 8*

5